# EUROPEAN PATENT SPECIFICATION

(11) **EP 2 682 096 B1**
(45) Date of publication and mention of the grant of the patent: **30.12.2015**
(21) Application number: 12850039.4
(22) Date of filing: 24.10.2012
(51) Int. Cl.: A61K 8/64, A61K 8/97, A61Q 5/12, C07K 2/00, C07K 1/12, A61K 8/42, A61K 8/65, A61K 8/88, A61K 47/48

(54) **COSMETIC BASE MATERIAL, AND COSMETIC CONTAINING SAID COSMETIC BASE MATERIAL**
KOSMETIKBASISMATERIAL UND KOSMETIKARTIKEL MIT DIESEM KOSMETIKBASISMATERIAL
MATÉRIAU DE BASE COSMÉTIQUE, ET COSMÉTIQUE CONTENANT LEDIT MATÉRIAU DE BASE COSMÉTIQUE

(30) Priority: 16.11.2011 JP 2011250217
(43) Date of publication of application: 08.01.2014
(73) Proprietor: Seiwa Kasei Company, Limited, Higashiosaka-shi Osaka 579-8004 (JP)
(72) Inventor: YOSHIOKA, Masato, Higashiosaka-shi Osaka 579-8004 (JP); KOBAYASHI, Eriko, Higashiosaka-shi Osaka 579-8004 (JP); KASAHARA, Yoshihito, Higashiosaka-shi Osaka 579-8004 (JP)
(74) Representative: Hamer, Christopher K.
(86) International application number: PCT/JP2012/077401
(87) International publication number: WO 2013/073350

(56) References cited:
- WO-A1-2012/140725
- DE-B- 1 238 158
- JP-A- S63 264 597
- JP-A- 2001 335 437
- JP-A- 2004 196 733
- JP-A- 2007 302 615
- JP-A- 2010 105 978
- JP-A- 2011 088 828
- JP-A- 2011 088 828
- US-A1- 2005 025 725

## Description

### Technical Field

The present invention relates to a cosmetic base material and cosmetics containing the cosmetic base material. Particularly, it relates to a cosmetic base material which manifests excellent conditioning actions on hairs such as imparting hydrophobicity and smoothness to damaged hairs, improving the combability of the hairs and the like, when blended in a hair cosmetic such as a hair treatment preparation, and cosmetics containing the cosmetic base material.

### Background Art

On the surface of the healthy hair, a lipid layer called F-layer containing 18-methyl eicosanoic acid as the main component is present, and contributes significantly to the hydrophobicity, smoothness and combability of the hair. However, the hair damaged by a chemical treatment with a permanent wave agent, a hair color agent and the like or by a physical treatment such as brushing and the like shows a decrease in the hydrophobicity and smoothness of the surface of the hair, resulting in poor combability. Then, cosmetic base materials having various hair conditioning actions have been developed and blended in a cosmetic for the purpose of improving these lowered properties.

As the cosmetic base material having a hair conditioning action, peptides and derivatives thereof are frequently used owing to strong adsorption force to hairs, and already blended in hair cosmetics such as hair treatment preparations, shampoos and the like. Of them, especially, peptides obtained by hydrolyzing proteins (protein hydrolysates) and derivatives thereof have been extensively used (patent document 1).

As the peptide derivative, acyl peptides obtained by amide-bonding a peptide and a fatty acid have been developed as a cosmetic base material having an enhanced hair conditioning action of a peptide and blended in various kinds of hair cosmetics (patent document 2, etc.).

Particularly, an ion complex composed of a specific surfactant and a peptide or peptide derivative having an acidic amino acid as the constituent amino acid manifests strong adsorption force to the hair, hydrophobicizes the surface of the hair having a functional property lowered by a damage, and is excellent in an action of imparting flexibility, moisturing property, smoothness, gloss and the like to the hair. Then, a cosmetic base material composed of a peptide or peptide derivative forming the ion complex, and cosmetics containing the cosmetic base material have been developed (patent document 3).

The above-described cosmetic base material composed of a peptide or peptide derivative forming an ion complex with a surfactant is good in an ability of imparting hydrophobicity and smoothness to the damaged hair and thus gives good combability to the hair. However, its effect is not recognized as to recover the hair condition to the condition before being damaged and is not sufficient in improving combability.

### Prior technological document

### [Patent document]

Patent document 1: JP-A No. 2007-302615
Patent document 2: JP-A No. 2004-196733
Patent document 3: JP-A No. 2011-88828

### Summary of the Invention

### Problem to be Solved by the Invention

An object of the present invention is to provide a cosmetic base material capable of imparting hydrophobicity and smoothness and giving a good combability to a hair deprived of the F-layer due to damages, and having lowered hydrophobicity, smoothness and combability, by coating the surface of the hair with the cosmetic base material, and to provide cosmetics containing the cosmetic base material.

### Means for Solving the Problem

The present inventors have intensively studied to solve the above-described problem and resultantly found that a fatty acid amide amine salt of an acyl peptide obtained by neutralizing part or all of acidic functional groups of an acyl peptide with a specific fatty acid amide amine is excellent in an ability of imparting hydrophobicity and smoothness to hair and giving good combability to hair. Thus, the present invention has been completed based on this finding.

That is, the present invention provides; a cosmetic base material composed of a fatty acid amide amine salt of an acyl peptide obtained by neutralizing part or all of acidic functional groups of an acyl peptide in which a substituent represented by the following general formula (I) : 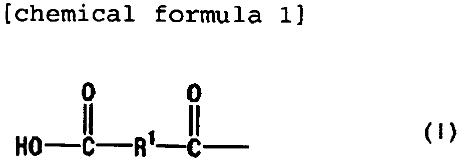 (wherein, R¹ represents -CH₂CH₂-, -CH=CH- or -C₆H₄-) is linked to an active amino group of a peptide, with an fatty acid amide amine represented by the following general formula (II) : 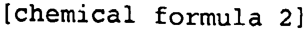 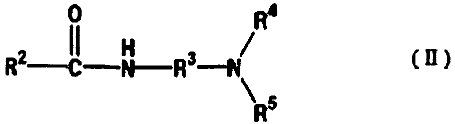 (wherein, R² represents a saturated or unsaturated linear hydrocarbon having 11 to 25 carbon atoms or a saturated or unsaturated cyclic hydrocarbon having 11 to 25 carbon atoms, R³ represents an alkylene group having 1 to 3 carbon atoms, and R⁴ and R⁵ represent each independently an alkyl group having 1 to 3 carbon atoms) as the basic invention. This invention is recognized as the invention according to Claim 1.

In the present invention, the acidic functional group denotes a carboxy group and a sulfo group. "All acidic functional groups" means acidic functional groups including a carboxy group and a sulfo group of a side chain of an acidic amino acid, an end carboxy group of the peptide main chain and a carboxy group in a substituent represented by the above-described general formula (I) linked to an active amino group of a peptide, and the sum of these acidic functional groups is defined as 100% by mol.

In the present invention, a fatty acid amide amine salt of an acyl peptide obtained by neutralizing part or all of acidic functional groups of an acyl peptide in which a substituent represented by the above-described general formula (I) is linked to an active amino group of a peptide, with a fatty acid amide amine represented by the above-described general formula (II) is a basic technology. Thus, the effect of the present invention is manifested more remarkably when the peptide has an acidic functional group not only at the end of the main chain but also at intermediate parts thereof (namely, parts other than end). That is, if the peptide has an acidic functional group at a side chain in addition to a carboxy group at the end of the main chain, which the peptide usually has, a salt with a fatty acid amide amine is formed in larger amount. Then, in this application, the cosmetic base material according to Claim 1 wherein the peptide has an acidic amino acid as the constituent amino acid is provided as an invention according to Claim 2.

As the peptide, those which are industrially relatively easily available are preferable. Then, in the present invention, a protein hydrolysate or a polyacidic amino acid is preferable as the peptide.

Of the above-described protein hydrolysate, plant protein hydrolysates, a keratin hydrolysate and a casein hydrolysate containing a large amount of acidic amino acids are preferable. Of the above-described polyacidic amino acid, polyaspartic acid and polyglutamic acid are easily-available. Hence, they are preferable for forming a fatty acid amide amine salt of an acyl peptide, the basic technology of the present invention.

Based on the above-described matters, the cosmetic base material according to Claim 1 or 2 wherein the peptide is a plant protein hydrolysate, a keratin hydrolysate, a casein hydrolysate, polyaspartic acid or polyglutamic acid is provided as an invention according to Claim 3

Of plant protein hydrolysates, a pea protein hydrolysate obtained by hydrolyzing a pea protein is more preferable from the standpoint of quality and safeness as a cosmetic base material. Thus, the cosmetic base material according to Claim 3 wherein the plant protein hydrolysate is a pea protein hydrolysate is provided as an invention according to Claim 4.

Though the suitable range of the degree of amino acid polymerization of a peptide varies depending on the kind of cosmetics in which the peptide is blended, the average degree of amino acid polymerization is preferably 2 to 100 in view of the stability of cosmetics when the peptide is blended in the cosmetic and the adsorption ability of the cosmetic base material of the present invention to the hair. Then, in the present application, the cosmetic base material according to any one of Claims 1 to 4 wherein the average degree of amino acid polymerization of a peptide part is 2 to 100 is provided as an invention according to Claim 5.

The cosmetic base material according to Claim 1 wherein the acyl peptide for forming a salt with a fatty acid amide amine is an acyl peptide represented by the following general formula (III):

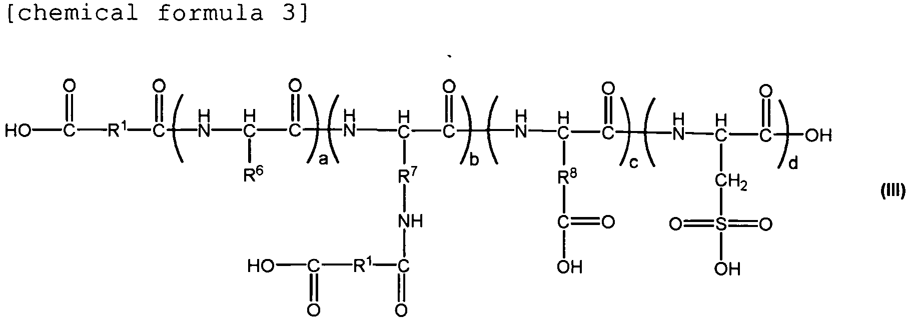

(wherein, R¹ is the same as in the above-described general formula (I), R⁶ represents a residue of a side chain at a neutral amino acid, R⁷ represents a residue of a side chain excluding an amino group of a basic amino acid, R⁸ represents an alkylene group having 1 to 2 carbon atoms, a, b, c and d represent the number of each amino acid, a+b+c+d representing the degree of amino acid polymerization, wherein a+b+c+d is 2 or more and c+d is 1 or more, and a, b, c and d represent only the number of an amino acid, and do not represent the order of amino acid sequence)
is provided as an invention according to Claim 6.

The cosmetic base material of the present invention has a feature of imparting hydrophobicity and smoothness to hair and improving combability of the hair having lowered hydrophobicity, smoothness and combability due to damages. Thus, in the instant application, cosmetics comprising the cosmetic base material according to any one of Claims 1 to 6 are provided as an invention according to Claim 7.

Since the cosmetic base material of the present invention has a property of improving hair condition as described above, the effect of the present invention is manifested remarkably particularly when blended in a hair cosmetic for improving hair condition such as a hair treatment preparation, a hair mist, and the like.

The invention according to Claim 8 is the cosmetic according to Claim 7 wherein the content of the above-described cosmetic base material is 0.1 to 10% by mass. Though the suitable range of the content of the cosmetic base material of the present invention in the cosmetic varies depending on the kind of the cosmetic, use mode thereof and the like, usually, 0.1 to 10% by mass with respect to the total mass of the cosmetic is preferable.

### Effect of the Invention

The cosmetic base material of the present invention is capable of imparting hydrophobicity and smoothness to the hair having lowered hydrophobicity, smoothness and combability due to damages, thereby improving the combability of the hair, and resultantly leading to recovery of the combability in the original healthy condition. Additionally, the combability of the healthy hair can be further improved. Therefore, the cosmetic base material is suitably used particularly in a hair cosmetic, and manifests the functions as described above. Further, the cosmetic base material of the present invention can also be blended in a skin cosmetic, thereby imparting softening and a moisturing property to dry skin.

### Modes for Carrying Out the Invention

Next, modes for carrying out the present invention will be illustrated. First, the peptide, the acyl peptide and the fatty acid amide amine used in constituting the cosmetic base material of the present invention are explained. Thereafter, the method of forming the fatty acid amide amine salt of an acyl peptide, the form of the cosmetic base material and the cosmetic in which the cosmetic base material is blended are explained.

### [Peptide]

The peptide used in constituting the cosmetic base material of the present invention has an active amino group, and a carboxy group or a sulfo group in the molecule. Their bonding sites may be a side chain of a peptide chain or the end thereof. Since peptides usually have an active amino group and have a carboxy group at the end of the peptide main chain, almost all peptides can be used in the cosmetic base material of the present invention, and the peptide may be a naturally-derived one, or one obtained by synthesis or fermentation.

As the above-described peptide, peptides having an acidic amino acid as the constituent amino acid are preferable. Since an acidic amino acid has a carboxy group or a sulfo group as a functional group, it tends to form a salt with many kinds of fatty acid amide amines to manifest the effect of the present invention. The peptide having an acidic amino acid as the constituent amino acid includes, for example, peptides represented by the following general formula (IV): 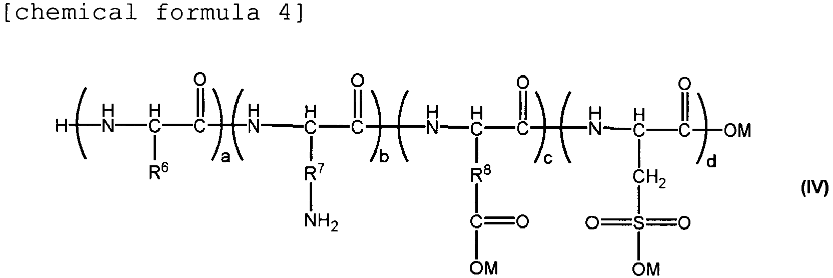 (in the formula (IV), R⁶ represents a residue of a side chain of a neutral amino acid, R⁷ represents a residue of a side chain excluding an amino group of a basic amino acid, R⁸ represents an alkylene group having 1 to 2 carbon atoms. M represents a hydrogen atom, an alkali metal, an alkaline earth metal and/or magnesium, a, b, c and d represent the number of each amino acid, a+b+c+d representing the degree of amino acid polymerization, wherein a+b+c+d is 2 or more and c+d is 1 or more, a, b, c and d represent only the number of an amino acid, and do not represent the order of amino acid sequence; a, b, c, d, a+b+c+d and c+d are theoretically an integer, however, since a peptide is often obtained as a mixture of those having different molecular weight, thus, these values denote an average value, and usually represented by a number other than an integer in many cases) and polyacidic amino acids such as polyaspartic acid, polyglutamic acid and the like represented by the following general formula (V): 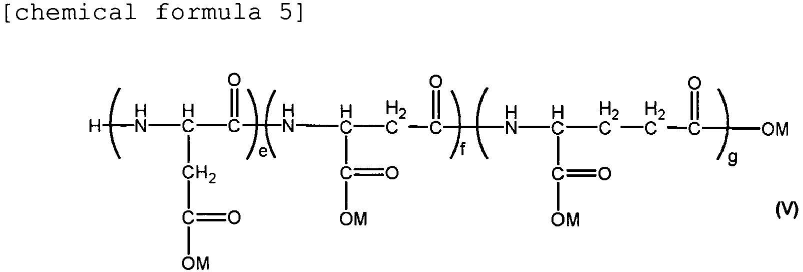 (in the formula (V), M represents a hydrogen atom, an alkali metal, an alkaline earth metal and/or magnesium, e, f and g represent the number of each amino acid, and e+f+g represents the degree of amino acid polymerization, wherein e+f+g is 2 or more, and e, f and g represent only the number of an amino acid, and do not represent the order of amino acid sequence; e, f, g and e+f+g are theoretically an integer, however, since a peptide is often obtained as a mixture of those having different molecular weights, thus, these values denote an average value, and usually represented by a number other than an integer in many cases.).

As the peptide, protein hydrolysates obtained by hydrolyzing a protein are preferable in view of industrially easy availability and safeness in blending the cosmetic base material of the present invention in cosmetics.

The above-described protein hydrolysate is obtained by partially hydrolyzing a protein with an acid, an alkali, an enzyme or a combination thereof. Its protein source includes animal proteins, plant proteins, microorganism-derived proteins and the like. The animal protein includes, for example, collagens (including also its denatured material: gelatin), keratin, fibroin, sericin, casein, conchiolin, elastin, protamine, yolk proteins and egg albumen proteins of chicken and the like. The plant protein includes, for example, proteins contained in soybean, wheat, rice (rice bran), sesame, pea, corn, tubers and roots of potatoes and the like. The microorganism-derived protein includes, for example, yeast proteins separated from saccharomyces, candida and endomycopsis yeast fungi or yeast fungi so called beer yeast and sake yeast, proteins separated from kinds of mushrooms (basidiomycete) and chlorella, spirulina proteins derived from seaweed, and the like.

Since the cosmetic base material of the present invention contains a fatty acid amide amine salt of an acyl peptide (in the present invention, the acyl peptide denotes a peptide having a carboxy group introduced by linking a substituent represented by the general formula (I) to an active amino group of a peptide) as the basic technology, if the acyl peptide has a larger amount of acidic functional groups forming a salt with a fatty acid amide amine, the effect of the present invention is manifested more remarkably. Then, it is preferable that the peptide part of acyl peptide contains an acidic amino acid as the constituent amino acid. Among the above-described proteins, plant proteins, keratin and casein are suitable as the protein source for forming a fatty acid amide amine salt of an acyl peptide in the cosmetic base material of the present invention because of high content of acidic amino acids, and plant protein hydrolysates, a keratin hydrolysate (hydrolyzed keratin) and a casein hydrolysates (hydrolyzed casein) obtained by hydrolyzing these proteins are suitable as the peptide used in constituting the cosmetic base material of the present invention. Of them, plant protein hydrolysates are particularly preferable.

Also polyacidic amino acids such as polyaspartic acid, polyglutamic acid and the like can be used as the peptide used in producing the cosmetic base material of the present invention, since these polyacidic amino acids are suitable for formation of a salt with a fatty acid amide amine.

Though peptides suitable for use in constituting the cosmetic base material of the present invention are described above, when industrially easy availability and high safeness in blending in cosmetics are taken into consideration in addition to the effect of the cosmetic base material of the present invention of improving the properties of the damaged hair, plant protein hydrolysates, a keratin hydrolysate, a casein hydrolysate, polyaspartic acid and polyglutamic acid are preferable. Of them, particularly plant protein hydrolysates are preferable.

Further, among plant protein hydrolysates, a pea protein hydrolysate is particularly preferable from the standpoint of quality as a cosmetic base material and safeness thereof.

The average polymerization degree of amino acid of a peptide is preferably in the range of 2 to 100. Therefore, in the general formula (IV), a+b+c+d is preferably 2 to 100, more preferably 3 to 50, further preferably 3 to 10. In the general formula (V), e+f+g is preferably 2 to 100, more preferably 3 to 50, further preferably 3 to 10.

The preferable range of the number of each amino acid unit constituting a peptide, that is, the number of each of a basic amino acid unit having an amino group at a side chain (amino acid unit affixed with b in the general formula (IV)), acidic amino acid units having a carboxy group at a side chain (amino acid unit affixed with c in the general formula (IV), amino acid unit affixed with e, f or g in the general formula (V)), an acidic amino acid unit having a sulfo group at a side chain (amino acid unit affixed with d in the general formula (IV)) and other amino acid units (amino acid units affixed with a in the general formula (IV)) in one molecule of the peptide varies depending on its application, raw material situations, the kind of each amino acid unit, etc., and is not particularly restricted. When, for example, 30% by mol or more of all acidic functional groups of an acyl peptide having a peptide skeleton represented by general formula (IV) form a salt with a fatty acid amide amine, c+d is preferably 1 to 10, more preferably 1 to 4. It is because the effect of the present invention is manifested easily and raw material peptides can be obtained easily.

### [Acyl peptide]

For use of the above-described peptide in the present invention, it is necessary to, first, prepare an acyl peptide in which a substituent represented by the above-described general formula (I) is linked to an active amino group of a peptide. In general, an acyl peptide used as a cosmetic base material is obtained by amide-bonding a linear or branched saturated or unsaturated fatty acid or resin acid having 8 to 32 carbon atoms to an amino group at the end of the peptide main chain and an amino group at a side chain of an amino acid. On the other hand, the acyl peptide used in the present invention is an acyl peptide in which a substituent represented by the above-described general formula (I) is linked to an active amino group of a peptide, and is characterized in that a carboxy group is newly introduced in the molecule. This carboxy group of the substituent is useful for formation of a salt with a fatty acid amide amine described later, and constitutes a factor for manifesting an excellent effect as a cosmetic base material.

The acyl peptide used in the present invention includes, for example, acyl peptides represented by the following general formula (VI).

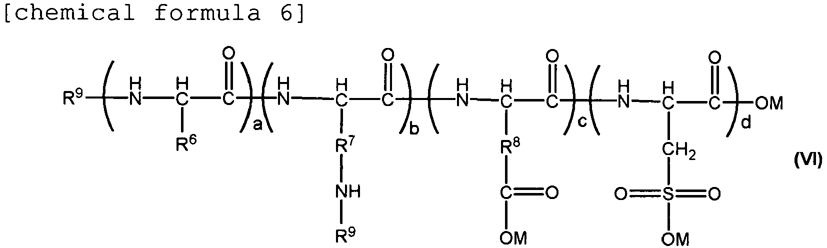

(in the general formula (VI), a, b, c, d, R⁶, R⁷, R⁸ and M are the same as in the above-described general formula (IV), R⁹ represents a substituent represented by the above-described general formula (I) or H, and two R⁹s may be the same or different, however, one of them should be a substituent represented by the above-described general formula (I)).

The active amino group of a peptide denotes an amino group at the end of the peptide and an amino group at a side chain of a basic amino acid constituting the peptide, and is an amino group showing nucleophilic reactivity. By reacting this active amino group showing nucleophilic reactivity with a dibasic organic acid anhydride and the like, an acyl peptide is obtained in which a substituent represented by the above-described general formula (I) is linked to an active amino group of a peptide.

As the dibasic organic acid anhydride, those capable of reacting with an active amino group of a peptide to introduce a carboxy group into the peptide can be used without particular restriction. When industrially easy availability and safeness are taken into consideration, for example, succinic anhydride, maleic anhydride and phthalic anhydride are preferable. Especially, succinic anhydride is more preferable since it is suitable for reaction with an active amino group of a peptide.

An acyl peptide obtained by reacting a peptide with succinic anhydride is called a succinylated peptide (acyl peptide in which a substituent represented by the above-described general formula (I) in which R¹ is -CH₂CH₂- is linked), an acyl peptide obtained by reacting a peptide with maleic anhydride is called a maleylated peptide (acyl peptide in which a substituent represented by the above-described general formula (I) in which R¹ is -CH=CH- is linked) and an acyl peptide obtained by reacting a peptide with phthalic anhydride is called a phthaloylated peptide (acyl peptide in which a substituent represented by the above-described general formula (I) in which R¹ is -C₆H₄- is linked), in some cases.

The reaction of a peptide with a dibasic organic acid anhydride can be carried out, for example, by a method described in JP63-264597A according to the application of the present applicant. That is, while stirring a peptide aqueous solution having a concentration of 5 to 60% by mass at pH 8 to 11 and a temperature of 30 to 60°C, a dibasic organic acid anhydride is bit by bit added to and dissolved in the peptide aqueous solution to cause reaction thereof, and in response to a decrease in pH during the reaction, an aqueous solution of an alkaline chemical such as sodium hydroxide, potassium hydroxide, lithium hydroxide and the like is added to maintain pH at 8 to 11. It is preferable that the necessary amount of the dibasic organic acid anhydride is added entirely and dissolved in the reaction system, then, the reaction is completed within 1 to 6 hours.

Depending on the reaction conditions, a ring opened product of a dibasic organic acid anhydride due to hydrolysis, that is, a dibasic organic acid such as succinic acid when succinic anhydride is used, maleic acid when maleic anhydride is used, and the like, is generated and thus an active amino group cannot be blocked sufficiently in some cases. Therefore, if necessary, amount of the dibasic organic acid anhydride used in the reaction may be increased.

The dibasic organic acid generated as a by-product can be removed by a means such as an ion exchange resin, an ion exchange membrane, ultrafiltration, electrodialysis and the like.

The cosmetic base material of the present invention is excellent in the effect of imparting hydrophobicity and smoothness to the hair and the effect of giving good combability as compared with the cosmetic base material described in the above-described patent document 3. The first reason is that the acyl peptide used in the present invention has a carboxy group in a substituent represented by the above-described general formula (I), and this carboxy group is ionically bonded to a fatty acid amide amine described later, to form a fatty acid amide amine salt of acyl peptide. The second reason is believed to be the following phenomenon. That is, usually, a peptide has an amino group and a carboxy group in the molecule, and the peptide tends to cause an intramolecular ionic bond owing to these two functional groups. However, in the peptide used in the cosmetic base material of the present invention, a substituent represented by the above-described general formula (I) is linked to its amino group. Therefore, an ionic bond between peptide molecules is not formed easily and a carboxy group of the acyl peptide tends to form an ionic bond with a fatty acid amide amine, resulting in formation of a fatty acid amide amine salt of acyl peptide showing a high effect of imparting hydrophobicity and smoothness to the hair and giving good combability.

### [Fatty acid amide amine]

The fatty acid amide amine used in the present invention is represented by the above-described general formula (II) wherein R² represents a saturated or unsaturated linear hydrocarbon having 11 to 25 carbon atoms or a saturated or unsaturated cyclic hydrocarbon having 11 to 25 carbon atoms, R³ represents an alkylene group having 1 to 3 carbon atoms and R⁴ and R⁵ represent each independently an alkyl group having 1 to 3 carbon atoms.

The fatty acid amide amine is obtained by amide-bonding an amino group of a dialkylaminoalkyleneamine to a carboxyl group of a fatty acid. An amide amine in which R² has a cyclic hydrocarbon is, for example, the one using rosin (pine resin) as a fatty acid. Rosin is a nonvolatile component of pine resin contained in a large amount in pinaceous plants, and constituted mainly of various isomers called also as a resin acid, and contains abietic acid, neoabietic acid, palustric acid, pimaric acid, isopimaric acid, dehydroabietic acid and the like. When stability and safeness as a cosmetic base material are taken in consideration, hydrogenated rosin obtained by hydrogenating rosin is preferably used.

As the fatty acid amide amine used in the present invention, any compound represented by the above-described general formula (II) may be used. Specific examples thereof include diethylaminoethylstearamide (that is, stearamidoethyl diethylamine), diethylaminoethylpalmitamide, diethylaminoethylbehenamide, diethylaminoethylmyristamide, diethylaminoethyllauramide, diethylaminoethylisostearamide, 3-dimethylaminopropylstearamide (that is, stearamidopropyl dimethylamine), 3-dimethylaminopropylpalmitamide, 3-dimethylaminopropylbehenamide, 3-dimethylaminopropylmyristamide, 3-dimethylaminopropyllauramide, 3-dimethylaminopropylisostearamide, diethylaminoethylresinamide, diethylaminoethyl-(hydrogenated-resinamide), 3-dimethylaminopropylresinamide, 3-dimethylaminopropyl-(hydrogenated-resinamide), and the like.

Of them, fatty acid amide amines which are industrially easily available and capable of sufficiently manifesting the effect of the present invention are, for example, diethylaminoethylstearamide, diethylaminoethylpalmitamide, diethylaminoethylbehenamide, diethylaminoethylisostearamide, diethylaminoethyl-(hydrogenated-resinamide) and the like. When stability and general versatility as a cosmetic base material are taken in consideration, diethylaminoethylstearamide and diethylaminoethylpalmitamide are more preferable, and diethylaminoethylcetostearamide (that is, cetearamidoethyl diethylamine) prepared by mixing these two fatty acid amide amines at a molar ratio of about 1:1 is particularly preferable.

When the cosmetic in which the cosmetic base material of the present invention is blended is a hair setting agent and a styling agent, it is particularly preferable to use diethylaminoethyl-(hydrogenated-resinamide) as the fatty acid amide amine since then, in addition to the basic effect of improving the properties of the damage hair of the present invention, a good hair setting effect is also manifested.

### [Method of forming fatty acid amide amine salt of acyl peptide]

In the cosmetic base material of the present invention, part or all of acidic functional groups of an acyl peptide to which a substituent represented by the above-described general formula (I) is linked are neutralized with a fatty acid amide amine represented by the above-described general formula (II), to form a fatty acid amide amine salt of acyl peptide. It is preferable that the neutralization ratio by a fatty acid amide amine represented by the general formula (II) (namely, neutralization ratio by a fatty acid amide amine represented by the general formula (II) with respect to all acidic functional groups of an acyl peptide) is higher. That is, a higher neutralization ratio is preferable since then the effect of imparting hydrophobicity and smoothness to the damaged hair and giving good combability of the hair is manifested more remarkably. In general, it is preferable that 30% by mol or more of all acidic functional groups of an acyl peptide are neutralized with a fatty acid amide amine represented by the general formula (II), particularly, 40% by mol or more of them are neutralized with a fatty acid amide amine represented by the general formula (II), though varying depending on the existence ratio of an acidic amino acid contained in the acyl peptide.

For forming a fatty acid amide amine salt of acyl peptide in the cosmetic base material of the present invention, for example, an acyl peptide is dissolved in water, a lower alcohol such as ethanol, isopropanol and the like, or a polyhydric alcohol such as propylene glycol, butylene glycol, glycerin and the like, and an acid chemical such as hydrochloric acid, sulfuric acid and the like is added to the acyl peptide solution to make its acidic value pH 4 or less, preferably 2.5 or less. Subsequently, this solution is demineralized by subjecting to an electrodialyzer or a dialysis tube. If the acyl peptide is insolubilized under acidic condition in this procedure, filtration or decantation is performed to recover the residue and the residue is washed thoroughly with water. By such an operation, a demineralized acyl peptide having a free acidic functional group (namely, -COOH or SO₃H) can be obtained. In the present invention, it is essential to use thus demineralized acyl peptide having a free acidic functional group.

Next, by adding a fatty acid amide amine represented by general formula (II) to the demineralized acyl peptide having a free acidic functional group, the acidic peptide solution having a free acidic functional group can be neutralized with the fatty acid amide amine, to obtain a cosmetic base material composed of a fatty acid amide amine salt of acyl peptide having high purity.

The fatty acid amide amine salt of acyl peptide formed as described above will be exemplified. Since a salt linkage (ionic bond) necessary for formation of a fatty acid amide amine salt of acyl peptide is generated at acidic functional groups of an acyl peptide, that is, at a carboxy group and a sulfo group, a case of generation of an ionic bond at a carboxy group and a case of generation of an ionic bond at a sulfo group will be separately explained.

When an ionic bond is formed between a carboxy group of an acyl peptide and a fatty acid amide amine represented by general formula (II), the fatty acid amide amine salt of acyl peptide is represented by the following general formula (VII). The peptide part of the acyl peptide is represented by the general formula (IV) or the general formula (V) as described above. In the example shown here, since an ionic bond to the fatty acid amide amine is formed at a carboxy group, only a carboxy group part is extracted and illustrated and the other part is represented by "acyl peptide body part". Namely, only a characteristic part of an ionic bond is illustrated, and the fatty acid amide amine salt of acyl peptide is abbreviated. An ionic bond between a carboxy group and a fatty acid amide amine is generated also at a carboxy group in a substituent represented by the above-described general formula (I), in addition to the peptide-derived carboxy group, and the fatty acid amide amine of acyl peptide salt formed by this ionic bond is a factor giving a sufficient effect to the cosmetic base material of the present invention.

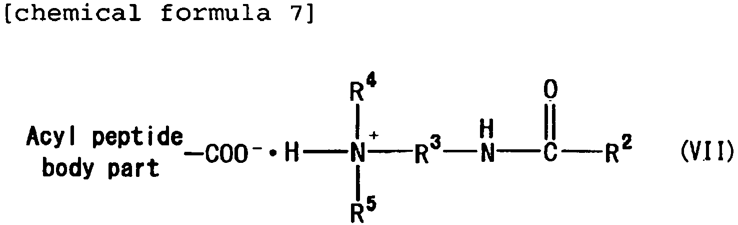

Next, when an acyl peptide is ionically bonded to a fatty acid amide amine represented by general formula (II) at a sulfo group, the acyl peptide fatty acid amide amine salt is represented by general formula (VIII). For this acyl peptide, only its sulfo group part is extracted and illustrated and the other part is represented by "acyl peptide body part" like the above-described case of a carboxy group.

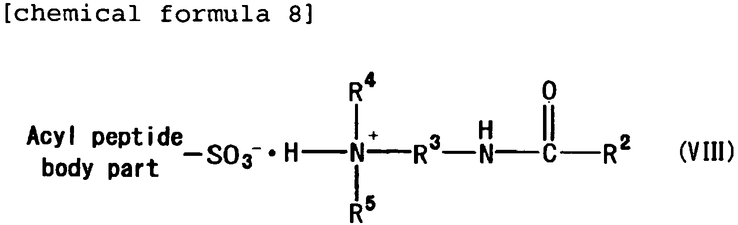

### [Form of cosmetic base material]

The cosmetic base material of the present invention is composed of a fatty acid amide amine salt of acyl peptide obtained by neutralizing part or all of acidic functional groups of an acyl peptide in which a substituent represented by the above-described general formula (I) is linked to an active amino group of a peptide with a fatty acid amide amine represented by the above-described general formula (II). This fatty acid amide amine salt of acyl peptide is dried to make a cosmetic base material in the form of solid. A solvent such as water, a lower alcohol, a polyhydric alcohol or the like may be optionally added to the cosmetic base material, to obtain a composition of the cosmetic base material in the form of paste or solution.

When an acyl peptide fatty acid amide amine salt is mixed with a water-containing ethanol solution or a water-containing glycerin solution, the whole solution is kept uniform and shows good stability. Thus it is preferable to prepare a water-containing ethanol solution or a water-containing glycerin solution of a fatty acid amide amine salt of acyl peptide, in providing a cosmetic base material in the form of solution. Particularly, a water-containing glycerin solution of a fatty acid amide amine salt of acyl peptide is excellent in stability and, when applied to the hair, shows a high effect of improving the combability of the hair.

The blending ratio of a fatty acid amide amine salt of acyl peptide, water and glycerin varies depending on the kind of the fatty acid amide amine salt of acyl peptide. It is preferable that fatty acid amide amine salt: water: glycerin is 5 to 80 : 5 to 75 : 15 to 60, particularly 7 to 60 : 5 to 73 : 20 to 50, especially 40 to 50 : 5 to 35 : 25 to 45 in terms of mass ratio, from the standpoint of productivity and stability as a cosmetic base material, in providing a fatty acid amide amine salt of acyl peptide in the form of solution.

### [Cosmetics in which cosmetic base material is blended]

The cosmetics in which the cosmetic base material of the present invention is blended includes, for example, hair cosmetics such as hair treatment preparations, hair conditioners, system treatment preparations, shampoos, hair mists, split end coats, hair creams, permanent wave first agent and second agent, curly hair relaxers, set lotions, hair waxes, styling agents, hair coloring agents, hair dyes, liquid styling agents, hair growth and hair nourishing agents, and the like. The cosmetic base material of the present invention may be blended in skin cosmetics such as cleansing creams, emollient creams, hand creams, after shaving lotions, shaving foams, face wash creams, face wash preparations, body shampoos, various soaps, hair removers, face packs, milky lotions, skin lotions, makeup cosmetics, sunscreen articles, and the like, and in this case, an effect of imparting flexibility, a moisturing property and affinity to the skin can be expected.

Though the preferable range of the amount of blending the cosmetic base material of the present invention in a cosmetic (content in cosmetic) varies depending on the kind of a cosmetic and is not particularly restricted, it is preferably 0.1 to 10% by mass in a cosmetic in many cases, particularly 0.3 to 5% by mass in a cosmetic in many cases. When the blending amount in a cosmetic is smaller than the above-described range, there is a possibility that the effect of imparting hydrophobicity and smoothness to the damage hair and improving hair combability cannot be manifested sufficiently. Even if the blending amount of the cosmetic base material of the present invention is larger than the above-described range, the correspondent improvement in the effect may not be obtained.

The component which can be blended together with the cosmetic base material of the present invention in the above-described cosmetic includes, for example, anionic surfactants, nonionic surfactants, ampholytic surfactants, cationic surfactants, synthetic polymers such as cationic polymers, ampholytic polymers, anionic polymers and the like, semi-synthetic polymers, animal and vegetable oils, hydrocarbons, ester oils, higher alcohols, amino acids, thickening agents, animal and plant extracts, silicones, preservatives, perfumes, protein hydrolysates obtained by hydrolyzing animal and plant-derived and microorganism-derived proteins, esterified derivatives of these protein hydrolysates, quaternary ammonium derivatives, silylated derivatives, acylated derivatives and the like, and salts thereof and the like. In addition to them, other components can be added appropriately in a range not deteriorating the property of the cosmetic base material of the present invention.

### EXAMPLES

The present invention will be illustrated specifically with reference to examples mentioned below, but the present invention is not limited only to these examples. In examples and the like, % is always by mass unless its unit is particularly described. Prior to descriptions of examples and the like, the method of measuring the amount of amino nitrogen and the total amount of nitrogen and the method of estimating the mole number of all acidic functional groups adopted in examples and the like will be explained.

### [Method of measuring amount of amino nitrogen and total amount of nitrogen]

Measurement of the amount of amino nitrogen in examples and the like is carried out by the van Slyke method. Measurement of the total amount of nitrogen is carried out by the improved Dumas method.

### [Method of estimating mole number of all acidic functional groups]

The mole number of all acidic functional groups of an acyl peptide is determined as described below. That is, it is supposed that the end carboxy group is present in equimolar amount to the mole number of amino nitrogen calculated from the amount of amino nitrogen of a peptide obtained by measuring by the van Slyke method. The existence mole number of acidic amino acids (glutamic acid, aspartic acid and cysteic acid) obtained by amino acid analysis is defined as the total mole number of carboxy group and sulfo group at side chains of a peptide. Sum of mole numbers of the end carboxy group, and carboxyl group and sulfo group at side chains, and further, carboxy group in the substituent represented by the above-described general formula (I) is estimated as the mole number of all acidic functional groups of an acyl peptide.

The degree of amino acid polymerization of a peptide used in examples (namely, a+b+c+d in the general formula (IV), or e+f+g in the general formula (V)) was calculated from the proportion of the total amount of nitrogen and the amount of amino nitrogen. Further, a:b:c:d and e+f:g were determined by amino acid analysis using an automated amino acid analyzer manufactured by Hitachi Ltd., and values of a, b, c, d, e+f and g were determined from the degree of amino acid polymerization. Protein hydrolysates used in examples were obtained by hydrolyzing respective protein sources by a known method using an acid, an alkali or an enzyme.

In Examples 1 to 9, specific examples of a fatty acid amide amine salt of acyl peptide are explained. In Comparative Examples 1 to 9 subsequent to Examples 1 to 9, specific examples of a fatty acid amide amine salt of a peptide or peptide derivative are explained, in Comparative Example 10, a succinylated pea peptide as one example of an acyl peptide is explained specifically, and in Comparative Example 11, a pea peptide as one example of a peptide is explained specifically.

### Example 1: Diethylaminoethylcetostearamide salt of succinylated pea peptide

To 50 g of an aqueous solution of a pea peptide having a concentration of 30% ("pea protein hydrolysate" is abbreviated as "pea peptide" as described previously) (in the general formula (IV), a=2.5, b=0.5, c=1.4, d=0.1, a+b+c+d=4.5) was added sodium hydroxide, to adjust pH to 10. This solution was heated to 55°C, and succinic anhydride was added in an amount corresponding to 1.5 equivalent with respect to an amino group of the pea peptide, and the mixture was stirred for 2 hours to perform the reaction.

Next, the reaction solution was treated by an electrodialyzer, thereby removing succinic acid generated by hydrolysis of succinic anhydride, to obtain a succinylated pea peptide solution.

To this succinylated pea peptide solution was added hydrochloric acid, to adjust pH to 2.5, then, demineralization thereof was performed by an electrodialyzer, to obtain a succinylated pea peptide solution having a free acidic functional group.

Subsequently, the above-described succinylated pea peptide solution was stirred with heating at 80°C, and diethylaminoethylcetostearamide was added in an amount corresponding to 60% by mol with respect to all acidic functional groups of the succinylated pea peptide, thereby neutralizing the succinylated pea peptide, to form a diethylaminoethylcetostearamide salt.

By the above-described operation, 98 g of a solution of the diethylaminoethylcetostearamide salt of succinylated pea peptide having a concentration of 40% was obtained. The neutralization ratio by diethylaminoethylcetostearamide in "diethylaminoethylcetostearamide salt of succinylated pea peptide" in this Example 1 was 60% by mol with respect to all acidic functional groups of the succinylated pea peptide.

### Example 2: Diethylaminoethylisostearamide salt of succinylated soybean peptide

To 50 g of an aqueous solution of a soybean peptide having a concentration of 30% ("soybean protein hydrolysate" is abbreviated as "soybean peptide" as described previously) (in the general formula (IV), a=1.7, b=0.3, c=1.2, d=0, a+b+c+d=3.2) was added sodium hydroxide to adjust pH to 10. This solution was heated to 55°C, and succinic anhydride was added in an amount corresponding to 1.5 equivalent with respect to an amino group of the soybean peptide, and the mixture was stirred for 2 hours to perform the reaction.

Next, the reaction solution was treated by an electrodialyzer, thereby removing succinic acid generated by hydrolysis of succinic anhydride, to obtain a succinylated soybean peptide solution.

To this succinylated soybean peptide solution was added hydrochloric acid, to adjust pH to 2.5, then, demineralization thereof was performed by an electrodialyzer, to obtain a succinylated soybean peptide solution having a free acidic functional group.

Subsequently, the above-described succinylated soybean peptide solution was stirred with heating at 80°C, and diethylaminoethylisostearamide was added in an amount corresponding to 100% by mol with respect to all acidic functional groups of the succinylated soybean peptide, thereby neutralizing the succinylated soybean peptide, to form a diethylaminoethylisostearamide salt.

By the above-described operation, 136 g of a solution of the diethylaminoethylisostearamide salt of succinylated soybean peptide having a concentration of 40% was obtained. The neutralization ratio by diethylaminoethylisostearamide in "diethylaminoethylisostearamide salt of succinylated soybean peptide" in this Example 2 was 100% by mol with respect to all acidic functional groups of the succinylated soybean peptide.

### Example 3: Diethylaminoethylbehenamide salt of succinylated sesame peptide

To 50 g of an aqueous solution of a sesame peptide having a concentration of 30% ("sesame protein hydrolysate" is abbreviated as "sesame peptide" as described previously) (in the general formula (IV), a=5.6, b=1.4, c=3.0, d=0, a+b+c+d=10.0) was added sodium hydroxide to adjust pH to 10. This solution was heated to 55°C, and succinic anhydride was added in an amount corresponding to 1.7 equivalent with respect to an amino group of the sesame peptide, and the mixture was stirred for 2 hours to perform the reaction.

Next, the reaction solution was treated by an electrodialyzer, thereby removing succinic acid generated by hydrolysis of succinic anhydride, to obtain a succinylated sesame peptide solution.

To this succinylated sesame peptide solution was added hydrochloric acid, to adjust pH to 2.5, then, demineralization thereof was performed by an electrodialyzer, to obtain a succinylated sesame peptide solution having a free acidic functional group.

Subsequently, the above-described succinylated sesame peptide solution was stirred with heating at 80°C, and diethylaminoethylbehenamide was added in an amount corresponding to 50% by mol with respect to all acidic functional groups of the succinylated sesame peptide, thereby neutralizing the succinylated sesame peptide, to form a diethylaminoethylbehenamide salt.

By the above-described operation, 69 g of a solution of the diethylaminoethylbehenamide salt of succinylated sesame peptide having a concentration of 40% was obtained. The neutralization ratio by diethylaminoethylbehenamide in "diethylaminoethylbehenamide salt of succinylated sesame peptide" in this Example 3 was 50% by mol with respect to all acidic functional groups of the succinylated sesame peptide.

### Example 4: Diethylaminoethylcetostearamide salt of succinylated keratin peptide

To 50 g of an aqueous solution of a keratin peptide having a concentration of 30% ("keratin protein hydrolysate" is abbreviated as "keratin peptide" as described previously) (in the general formula (IV), a=2.7, b=0.4, c=0.9, d=0.1, a+b+c+d=4.1) was added sodium hydroxide to adjust pH to 10. This solution was heated to 55°C, and succinic anhydride was added in an amount corresponding to 1.7 equivalent with respect to an amino group of the keratin peptide, and the mixture was stirred for 2 hours to perform the reaction.

Next, the reaction solution was treated by an electrodialyzer, thereby removing succinic acid generated by hydrolysis of succinic anhydride, to obtain a succinylated keratin peptide solution.

To this succinylated keratin peptide solution was added hydrochloric acid, to adjust pH to 2.0, then, demineralization thereof was performed by an electrodialyzer, to obtain a succinylated keratin peptide solution having a free acidic functional group.

Subsequently, the above-described succinylated keratin peptide solution was stirred with heating at 80°C, and diethylaminoethylcetostearamide was added in an amount corresponding to 70% by mol with respect to all acidic functional groups of the succinylated keratin peptide, thereby neutralizing the succinylated keratin peptide, to form a diethylaminoethylcetostearamide salt.

By the above-described operation, 97 g of a solution of the diethylaminoethylcetostearamide salt of succinylated keratin peptide having a concentration of 40% was obtained. The neutralization ratio by diethylaminoethylcetostearamide in "diethylaminoethylcetostearamide salt of succinylated keratin peptide" in this Example 4 was 70% by mol with respect to all acidic functional groups of the succinylated keratin peptide.

### Example 5: Diethylaminoethylpalmitamide salt of succinylated polyaspartic acid

To 50 g of an aqueous solution of a polyaspartic acid having a concentration of 30% (in the general formula (V), e+f=8.0, g=0) was added sodium hydroxide to adjust pH to 10. This solution was heated to 55°C, and succinic anhydride was added in an amount corresponding to 1.8 equivalent with respect to an amino group of the polyaspartic acid, and the mixture was stirred for 2 hours to perform the reaction.

Next, the reaction solution was treated by an electrodialyzer, thereby removing succinic acid generated by hydrolysis of succinic anhydride, to obtain a succinylated polyaspartic acid solution.

To this succinylated polyaspartic acid solution was added hydrochloric acid, to adjust pH to 2.0, then, demineralization thereof was performed by an electrodialyzer, to obtain a succinylated polyaspartic acid solution having a free acidic functional group.

Subsequently, the above-described succinylated polyaspartic acid solution was stirred with heating at 80°C, and diethylaminoethylpalmitamide was added in an amount corresponding to 40% by mol with respect to all acidic functional groups of the succinylated polyaspartic acid, thereby neutralizing the succinylated polyaspartic acid, to form a diethylaminoethylpalmitamide salt.

By the above-described operation, 76 g of a solution of the diethylaminoethylpalmitamide salt of succinylated polyaspartic acid having a concentration of 40% was obtained. The neutralization ratio by diethylaminoethylpalmitamide in "diethylaminoethylpalmitamide salt of succinylated polyaspartic acid" in this Example 5 was 40% by mol with respect to all acidic functional groups of the succinylated polyaspartic acid.

### Example 6: Diethylaminoethylisostearamide salt of maleylated wheat peptide

To 50 g of an aqueous solution of a wheat peptide having a concentration of 30% ("wheat protein hydrolysate" is abbreviated as "wheat peptide" as described previously) (in the general formula (IV), a=36.2, b=12.2, c=34.5, d=0.6, a+b+c+d=83.5) was added sodium hydroxide to adjust pH to 10. This solution was heated to 55°C, and maleic anhydride was added in an amount corresponding to 4.0 equivalents with respect to an amino group of the wheat peptide, and the mixture was stirred for 2 hours to perform the reaction.

Next, hydrochloric acid was added to the reaction solution to adjust pH to 2.0, thereby insolubilizing a maleylated wheat peptide. This insoluble material was washed with water to remove inorganic salts and maleic acid generated by hydrolysis of maleic anhydride, thereby obtaining a maleylated wheat peptide dispersion having a free acidic functional group.

Subsequently, the above-described maleylated wheat peptide dispersion was stirred with heating at 80°C, and diethylaminoethylisostearamide was added in an amount corresponding to 90% by mol with respect to all acidic functional groups of the maleylated wheat peptide, thereby neutralizing the maleylated wheat peptide, to form a diethylaminoethylisostearamide salt.

By the above-described operation, 158 g of a solution of the diethylaminoethylisostearamide salt of maleylated wheat peptide having a concentration of 40% was obtained. The neutralization ratio by diethylaminoethylisostearamide in "diethylaminoethylisostearamide salt of maleylated wheat peptide" in this Example 6 was 90% by mol with respect to all acidic functional groups of the maleylated wheat peptide.

### Example 7: 3-Dimethylaminopropylcetostearamide salt of maleylated polyglutamic acid

To 50 g of an aqueous solution of a polyglutamic acid having a concentration of 30% (in the general formula (V), e+f=0, g=20.0) was added sodium hydroxide to adjust pH to 10. This solution was heated to 55°C, and maleic anhydride was added in an amount corresponding to 4.0 equivalent with respect to an amino group of the polyglutamic acid, and the mixture was stirred for 2 hours to perform the reaction.

Next, the reaction solution was treated by an electrodialyzer, thereby removing maleic acid generated by hydrolysis of maleic anhydride to obtain a maleylated polyglutamic acid solution.

To this maleylated polyglutamic acid solution was added hydrochloric acid to adjust pH to 2.0, then, demineralization thereof was performed by an electrodialyzer, to obtain a maleylated polyglutamic acid solution having a free acidic functional group.

Subsequently, the above-described maleylated polyglutamic acid solution was stirred with heating at 80°C, and 3-dimethylaminopropylcetostearamide was added in an amount corresponding to 50% by mol with respect to all acidic functional groups of the maleylated polyglutamic acid, thereby neutralizing the maleylated polyglutamic acid, to form a 3-dimethylaminopropylcetostearamide salt.

By the above-described operation, 207 g of a solution of the 3-dimethylaminopropylcetostearamide salt of maleylated polyglutamic acid having a concentration of 40% was obtained. The neutralization ratio by 3-dimethylaminopropylcetostearamide in "3-dimethylaminopropylcetostearamide salt of maleylated polyglutamic acid" in this Example 7 was 50% by mol with respect to all acidic functional groups of the maleylated polyglutamic acid.

### Example 8: 3-Dimethylaminopropylisostearamide salt of phthaloylated rice peptide

To 50 g of an aqueous solution of a rice peptide having a concentration of 30% ("rice protein hydrolysate" is abbreviated as "rice peptide" as described previously) (in the general formula (IV), a=31.3, b=5.9, c=12.8, d=0.3, a+b+c+d=50.3) was added sodium hydroxide, to adjust pH to 10. This solution was heated to 55°C, and phthalic anhydride was added in an amount corresponding to 4.0 equivalent with respect to an amino group of the rice peptide, and the mixture was stirred for 2 hours to perform the reaction.

To this phthaloylated rice peptide solution was added hydrochloric acid to adjust pH to 2.0, and the phthaloylated rice peptide was insolubilized. The insoluble matter thus obtained was washed with water, thereby removing inorganic salts and phthalic acid generated by hydrolysis of phthalic anhydride to obtain a phthaloylated rice peptide dispersion having a free acidic functional group.

Subsequently, the above-described phthaloylated rice peptide dispersion was stirred with heating at 80°c, and 3-dimethylaminopropylisostearamide was added in an amount corresponding to 80% by mol with respect to all acidic functional groups of the phthaloylated rice peptide, thereby neutralizing the phthaloylated rice peptide, to form a 3-dimethylaminopropylisostearamide salt.

By the above-described operation, 107 g of a solution of the 3-dimethylaminopropylisostearamide salt of phthaloylated rice peptide having a concentration of 40% was obtained. The neutralization ratio of 3-dimethylaminopropylisostearamide in "3-dimethylaminopropylisostearamide salt of phthaloylated rice peptide" in this example 8 was 80% by mol with respect to all acidic functional groups of the phthaloylated rice peptide.

### Example 9: Diethylaminoethyl-(hydrogenated-resinamide) salt of phthaloylated casein peptide

To 50 g of an aqueous solution of a casein peptide having a concentration of 30% ("casein protein hydrolysate" is abbreviated as "casein peptide" as described previously) (in the general formula (IV), a=3.6, b=0.8, c=1.8, d=0, a+b+c+d=6.2) was added sodium hydroxide to adjust pH to 10. This solution was heated to 55°C, and phthalic anhydride was added in an amount corresponding to 3.0 equivalent with respect to an amino group of the casein peptide, and the mixture was stirred for 2 hours to perform the reaction.

Next, the reaction solution was treated by an electrodialyzer, thereby removing phthalic acid generated by hydrolysis of phthalic anhydride, to obtain a phthaloylated casein peptide solution.

To this phthaloylated casein peptide solution was added hydrochloric acid to adjust pH to 2 .0, then, demineralization thereof was performed by an electrodialyzer, to obtain a phthaloylated casein peptide solution having a free acidic functional group.

Subsequently, the above-described phthaloylated casein peptide solution was stirred with heating at 80°C, and diethylaminoethyl-(hydrogenated-resinamide) was added in an amount corresponding to 50% by mol with respect to all acidic functional groups of the phthaloylated casein peptide, thereby neutralizing the phthaloylated casein peptide, to form diethylaminoethyl-(hydrogenated-resinamide) salt.

By the above-described operation, 97 g of a solution of the diethylaminoethyl-(hydrogenated-resinamide) salt of phthaloylated casein peptide having a concentration of 40% was obtained. The neutralization ratio by diethylaminoethyl-(hydrogenated-resinamide) in "diethylaminoethyl-(hydrogenated-resinamide) salt of phthaloylated casein peptide" in this Example 9 was 50% by mol with respect to all acidic functional groups of the phthaloylated casein peptide.

### [Preparation of comparative example]

### Comparative Examples 1 to 9

Fatty acid amide amine salt of peptide or peptide derivative

Salts with a fatty acid amide amine were prepared, as Comparative Examples 1 to 9, by using peptides or peptide derivatives shown below instead of the acyl peptide (succinylated peptide, maleylated peptide, phthaloylated peptide) used in Examples 1 to 9. The degree of amino acid polymerization of peptide used, the kind of a fatty acid amide amine and the amount of a fatty acid amide amine added were the same as in the example corresponding to each peptide.
Comparative Example 1: pea peptide
Comparative Example 2: lauroyl soybean peptide
Comparative Example 3: silyl sesame peptide (that is, (dihydroxymethylsilylpropoxy)hydroxypropyl sesame peptide)
Comparative Example 4: keratin peptide
Comparative Example 5: polyaspartic acid
Comparative Example 6: wheat peptide
Comparative Example 7: polyglutamic acid
Comparative Example 8: glycerylated rice peptide
Comparative Example 9: N-[2-hydroxy-3-(trimethylammonio)propyl] casein peptide chloride
Comparative Example 10: Succinylated pea peptide

The succinylated pea peptide used in Example 1 was used without forming a salt with a fatty acid amide amine, as Comparative Example 10.

### Comparative Example 11: Pea peptide

The pea peptide used in Example 1 was used without succinylation and without forming a salt with a fatty acid amide amine, as Comparative Example 11.

### [Preparation of hair sample for evaluation]

### Healthy hair:

hair treated by the following treatment (a), then, treated only by the following treatment (d). (Control Example 1)

### Damaged hair:

hair treated by the following treatments (a) to (e).

### (Control Example 2)

### Sample hair:

hair treated by the following treatments (A) to (C).

### (Examples 1 to 9 and Comparative Examples 1 to 11)

The above-described sample hair denotes the hair treated by using the fatty acid amide amine salt of acyl peptide of Examples 1 to 9, the fatty acid amide amine salts of peptide or peptide derivative of Comparative Examples 1 to 9, the succinylated pea peptide of Comparative Example 10 or the pea peptide of Comparative Example 11.
(a) Hairs of an Asian are washed with a 2% sodium polyoxyethylene (3) lauryl ether sulfate aqueous solution, to remove stains and oils adhered to the hair surface.
(b) The sample is immersed in a mixed solution of 6% hydrogen peroxide water and 2% ammonia water at 30°C for 30 minutes, to bleach the sample.
(c) The sample is immersed in a pH 3.0 citric acid buffer solution for 5 minutes.
(d) The sample is washed with running water, and dried by a drier.
(e) The treatments (b) to (d) are repeated 5 times.
   (A) The damaged hairs obtained by the above-described treatments (a) to (e) are shaken at 40°C for 10 minutes in an aqueous solution of each sample of Examples 1 to 9 and Comparative Examples 1 to 11 having a solid concentration of 0.3%.
   (B) The sample is washed with running water, then, dried by a drier.
   (C) The treatments (A) to (B) are repeated 5 times.

### [Evaluation]

The smoothness, the hydrophobicity and the combability are evaluated using the hair samples for evaluation obtained by performing the above-described treatments, that is, the healthy hair, the damaged hairs and the sample hairs treated by cosmetic base materials obtained in Examples 1 to 9 and Comparative Examples 1 to 11.

### 1. Evaluation of smoothness

The smoothness of the hair surface is evaluated by measuring the dynamic friction coefficient using a friction feeling tester (manufactured by KATO TECH Co., Ltd.). Twenty hairs are arranged and fixed on a slide glass, a sensor is slid from the root of the hair toward the hair tip, and the average friction coefficient (MIU(×10⁻¹)) is measured. Smaller value of the average friction coefficient indicates smoother hair surface.

### 2. Evaluation of hydrophobicity

A water drop (1.5 µL) is dropped on one hair, and the contact angle (°) formed between the hair and the water drop is measured. This procedure is conducted 10 times for each hair, and the average value thereof is determined. Larger contact angle indicates that the hair surface keeps higher hydrophobicity.

### 3. Evaluation of combability

The combability of a hair bundle made of the above-described treated hairs is evaluated using a combing tester (manufactured by Techno Hashimoto). Combing is conducted 10 times using the above-described combing tester for one hair bundle, and the average load value generated in this procedure is measured and compared, to evaluate combability. Three hair bundles are prepared for each subject, combing is repeated each 10 times, and the average value thereof is calculated. Smaller value of the average load value(N) indicates better combability of hair.

The evaluation results of these "smoothness", "hydrophobicity" and "combability" are shown in Table 1. Table 1 shows also sample names of Examples 1 to 9 and Comparative Examples 1 to 11. However, the names of fatty acid amide amine salts of acyl peptide of Examples 1 to 9 and fatty acid amide amine salts of peptide or peptide derivative of Comparative Examples 1 to 9 are long and it is difficult to describe these names entirely in Table 1. Therefore, fatty acid amide amine salt parts thereof are abbreviated as described below.
CSEE salt: diethylaminoethylcetostearamide salt
ISEE salt: diethylaminoethylisostearamide salt
BHEE salt: diethylaminoethylbehenamide salt
PLEE salt: diethylaminoethylpalmitamide salt
CSMP salt: 3-dimethylaminopropylcetostearamide salt
ISMP salt: 3-dimethylaminopropylisostearamide salt
HREE salt: diethylaminoethyl-(hydrogenated-resinamide) salt

**[Table 1]**

| | | Smoothness | Hydrophobicity | Combability |
|---|---|---|---|---|
| | | Average friction coefficient | Contact angle (°) | Average load value (N) |
| Control Example 1 | Healthy hair | 0.92 | 114.6 | 12.10 |
| Control Example 2 | Damaged hair | 1.05 | 86.2 | 16.60 |
| Example 1 | CSEE salt of succinylated pea peptide | 0.75 | 99.9 | 9.50 |
| Example 2 | ISEE salt of succinylated soybean peptide | 0.70 | 105.4 | 9.00 |
| Example 3 | BHEE salt of succinylated sesame peptide | 0.77 | 99.4 | 9.55 |
| Example 4 | CSEE salt of succinylated keratin peptide | 0.73 | 100.4 | 9.02 |
| Example 5 | PLEE salt of succinylated polyaspartic acid | 0.76 | 99.0 | 9.20 |
| Example 6 | ISEE salt of maleylated wheat peptide | 0.78 | 98.1 | 9.52 |
| Example 7 | CSMP salt of maleylated polyglutamic acid | 0.75 | 99.2 | 9.28 |
| Example 8 | ISMP salt of phthaloylated rice peptide | 0.79 | 98.0 | 9.62 |
| Example 9 | HREE salt of phthaloylated casein peptide | 0.78 | 98.5 | 9.99 |
| Comparative Example 1 | CSEE salt of pea peptide | 0.91 | 92.0 | 13.00 |
| Comparative Example 2 | ISEE salt of lauroyl soybean peptide | 0.82 | 96.1 | 12.50 |
| Comparative Example 3 | BHEE salt of silyl sesame peptide | 0.90 | 94.8 | 13.02 |
| Comparative Example 4 | CSEE salt of keratin peptide | 0.85 | 92.0 | 14.20 |
| Comparative Example 5 | PLEE salt of polyaspartic acid | 0.84 | 95.8 | 13.53 |
| Comparative Example 6 | ISEE salt of wheat peptide | 0.82 | 94.4 | 14.10 |
| Comparative Example 7 | CSMP salt of polyglutamic acid | 0.88 | 92.0 | 14.50 |
| Comparative Example 8 | ISMP salt of glycerylated rice peptide | 0.85 | 95.9 | 12.99 |
| Comparative Example 9 | HREE salt of trimethylammoniumated casein peptide | 0.90 | 90.4 | 14.80 |
| Comparative Example 10 | Succinylated pea peptide | 1.01 | 86.0 | 15.01 |
| Comparative Example 11 | pea peptide | 0.97 | 86.2 | 14.99 |

As shown in Table 1, it is understood that hairs treated with the fatty acid amide amine salt of acyl peptide of Examples 1-9 show a smaller average friction coefficient in the evaluation of a smoothness, larger contact angle in the evaluation of hydrophobicity and a smaller average load value in the evaluation of combability as compared with hairs treated with fatty acid amide amine salts of the peptide or peptide derivative of Comparative Examples 1 to 9, hairs treated with the succinylated pea peptide of Comparative Example 10 and hairs treated with the pea peptide of Comparative Example 11. That is, the fatty acid amide amine salt of acyl peptide of Examples 1 to 9 is excellent in effects of imparting hydrophobicity and smoothness to the damage hair and improving combability.

Also hairs treated with the fatty acid amide amine salts of peptide or peptide derivative of Comparative Examples 1 to 9 show a smaller average friction coefficient in the evaluation of a smoothness, a larger contact angle in the evaluation of hydrophobicity and a smaller average load value in the evaluation of combability as compared with the damaged hairs shown in Control Example 2, hairs treated with the succinylated pea peptide of Comparative Example 10, hairs treated with the pea peptide of Comparative Example 11 and the like. That is, the fatty acid amide amine salts of peptide or peptide derivative of Comparative Examples 1 to 9 have effects of imparting hydrophobicity and smoothness to the damaged hair and improving combability. However, those effects are smaller as compared with the fatty acid amide amine salt of acyl peptide of Examples 1 to 9.

That is, for example, if Example 1 and Comparative Example 1 both using a pea peptide are compared, hairs treated with the diethylaminoethylcetostearamide salt of succinylated pea peptide of Example 1 (in Table 1, depicted as "CSEE salt") have an average friction coefficient of 0.75, while hairs treated with the diethylaminoethylcetostearamide salt of pea peptide of Comparative Example 1 (in Table 1, depicted as "CSEE salt") have an average friction coefficient of 0.91, as shown in Table 1. That is the average friction coefficient indicative of smoothness is smaller in Example 1 by as much as 0.16 than in Comparative Example 1. With respect to other Examples and Comparative examples, similarly, comparing an Example and a Comparative example using the same peptide, the average friction coefficient is smaller in Example than in Comparative Example. It is understood from this that the cosmetic base material of the present invention composed of a fatty acid amide amine salt of acyl peptide has a higher effect of imparting smoothness to the damaged hair.

Also regarding the contact angle, hairs treated with the diethylaminoethylcetostearamide salt of succinylated pea peptide of Example 1 have a contact angle of 99.9°, while hairs treated with the diethylaminoethylcetostearamide salt of pea peptide of Comparative Example 1 have a contact angle of 92.0°, that is, the contact angle indicative of hydrophobicity is larger in Example 1 by as much as 7.9° than in Comparative Example 1. With respect to other Examples and Comparative examples, similarly, comparing an Example and a Comparative example using the same peptide, the contact angle is larger in Example than in Comparative Example. It is understood from this that the cosmetic base material of the present invention composed of a fatty acid amide amine salt of acyl peptide has a higher effect of imparting hydrophobicity to the damaged hair.

Further, also regarding the combability, hairs treated with the diethylaminoethylcetostearamide salt of succinylated pea peptide of Example 1 have an average load value of 9.50 N, while hairs treated with the diethylaminoethylcetostearamide salt of pea peptide of Comparative Example 1 have an average load value of 13.00 N, that is, the average load value indicative of combability is smaller in Example 1 by as much as 3.5 N than in Comparative Example 1. With respect to other Examples and Comparative examples, similarly, comparing an Example and a Comparative example using the same peptide, the average load value is smaller in Example than in Comparative Example. It is understood from this that the cosmetic base material of the present invention composed of an acyl peptide fatty acid amide amine salt has a higher effect of improving a hair combability.

Further, hairs treated with the fatty acid amide amine salts of peptide or peptide derivative of Comparative Example 1 to 9 have a smaller average load value indicative of combability than the damaged hair shown in Control Example 2, however have a larger average load value as compared with the healthy hair shown in Control Example 1. That is, the fatty acid amide amine salts of peptide or peptide derivative of Comparative Example 1 to 9 cannot improve combability more than the healthy hair before being damaged. In contrast, hairs treated with the fatty acid amide amine salt of acyl peptide of Example 1 have a smaller average load value than the healthy hair shown in Control Example 1. That is, it is understood that the fatty acid amide amine salt of acyl peptide of Example 1 can improve the combability of the damaged hair more than the condition before being damaged, thus, can remarkably improve hair combability.

Further, when hairs treated with the succinylated pea peptide of Comparative Example 10 and hairs treated with the pea peptide of Comparative Example 11 are compared, there is no significant difference in any of the average friction coefficient, the contact angle and the average load value, however, when hairs treated with the diethylaminoethylcetostearamide salt of succinylated pea peptide of Example 1 and hairs treated with the diethylaminoethylcetostearamide salt of pea peptide of Comparative Example 1 are compared, there is a significant different between them in all of the average friction coefficient, the contact angle and the average load value. That is, the cosmetic base material of the present invention composed of a fatty acid amide amine salt of acyl peptide shows synergistically an effect by an acylation of linking a substituent represented by the general formula (I) to an active amino group of a peptide and an effect by formation of a fatty acid amide amine salt of acyl peptide.

### Examples 10-11 and Comparative Examples 12-13: Hair conditioner

Hair treatment preparations having compositions shown in Table 2 were prepared using each of the diethylaminoethylcetostearamide salt of succinylated pea peptide of Example 1, the diethylaminoethylcetostearamide salt of succinylated keratin peptide of Example 4, the diethylaminoethylcetostearamide salt of pea peptide of Comparative Example 1 and the diethylaminoethylcetostearamide salt of keratin peptide of Comparative Example 4, as Examples 10-11 and Comparative Examples 12-13. In Table 2, the amount of each component is shown in mass part.

**[Table 2]**

| Ingredients | Example 10 | Example 11 | Comparative Example 12 | Comparative Example 13 |
|---|---|---|---|---|
| Diethylaminoethylcetostearamide salt of succinylated pea peptide of Example 1 | 1.0 | - | - | - |
| Diethylaminoethylcetostearamide salt of succinylated keratin peptide of Example 4 | - | 5.0 | - | - |
| Diethylaminoethylcetostearamide salt of pea peptide of Comparative Example 1 | - | - | 1.0 | - |
| Diethylaminoethylcetostearamide salt of keratin peptide of Comparative Example 4 | - | - | - | 5.0 |
| Stearyl trimethyl ammonium chloride | 0.4 | 0.4 | 0.4 | 0.4 |
| Cetostearyl alcohol | 3.0 | 3.0 | 3.0 | 3.0 |
| Polyoxyethylene (20) stearyl ether | 1.0 | 1.0 | 1.0 | 1.0 |
| Glycerin | 1.8 | 1.8 | 1.8 | 1.8 |
| Glyceryl tri-2-ethyl hexanoate | 1.5 | 1.5 | 1.5 | 1.5 |
| Phenoxyethanol | 0.3 | 0.3 | 0.3 | 0.3 |
| Water | Amount to be a total of 100 | | | |

The hair conditioners of Examples 10-11 and Comparative Examples 12-13 were coated each in an amount of 1 g on 2 g of the above-described damaged hairs, and the hairs were washed with flowing water. Thereafter, the smoothness, the hydrophobicity and the combability were evaluated in the same manners as described above. The results are shown in Table 3.

**[Table 3]**

| | | Smoothness | Hydrophobicity | Combability |
|---|---|---|---|---|
| | | Average friction coefficient | Contact angle (°) | Average load value (N) |
| Example 10 | Diethylaminoethylcetostearamide salt of succinylated pea peptide of Example 1 | 0.78 | 95.0 | 9.98 |
| Example 11 | Diethylaminoethyl-cetostearamide salt of succinylated keratin peptide of Example 4 | 0.73 | 98.8 | 9.63 |
| Comparative Example 12 | Diethylaminoethylcetostearamide salt of pea peptide of Comparative Example 1 | 0.88 | 89.7 | 12.98 |
| Comparative Example 13 | Diethylaminoethylcetostearamide salt of keratin peptide of Comparative Example 4 | 0.83 | 91.0 | 12.20 |

As shown in Table 3, hairs treated with the hair conditioner of the examples 10 to 11 show a larger contact angle, a smaller average friction coefficient and a smaller average load value as compared with hairs treated with the hair conditioner of the comparative examples 12 to 13. It is understood that the hair conditioner of the examples 10 to 11 shows a higher effect of imparting hydrophobicity and smoothness to the damaged hair and improving combability than the hair conditioner of the comparative examples 12 to 13.

### Examples 12-13 and Comparative Examples 14-15: Hair mist

Hair mists having compositions shown in Table 4 were prepared using each of the diethylaminoethylpalmitamide salt of succinylated polyaspartic acid of example 5, the diethylaminoethylisostearamide salt of maleylated wheat peptide of example 6, the diethylaminoethylpalmitamide salt of polyaspartic acid of comparative example 5 and the diethylaminoethylisostearamide salt of wheat peptide of Comparative Example 6, as Examples 12-13 and Comparative Examples 14-15. In Table 4, the amount of each component is shown in mass part.

**[Table 4]**

| Ingredients | Example 12 | Example 13 | Comparative Example 14 | Comparative Example 15 |
|---|---|---|---|---|
| Diethylaminoethylpalmitamide salt of Succinylated polyaspartic acid of example 5 | 0.1 | - | - | - |
| Diethylamiswethyl-isostearamide salt of maleylated wheat peptide of example 6 | - | 0.5 | - | - |
| Diethylaminoethylpalmitamide salt of polyaspartic acid of comparative example 5 | - | - | 0.1 | - |
| Diethylaminoethylisostearamide salt of wheat peptide of comparative example 6 | - | - | - | 0.5 |
| Polyoxyethylene (20) stearyl ether | 0.3 | 0.3 | 0.3 | 0.3 |
| 1,3-butyleneglycol | 2.0 | 2.0 | 2.0 | 2.0 |
| Phenoxyethanol | 0.5 | 0.5 | 0.5 | 0.5 |
| Ethanol | 5.0 | 5.0 | 5.0 | 5.0 |
| Water | Amount to be a total of 100 | | | |

The hair mists of Examples 12-13 and Comparative Examples 14-15 were coated each in an amount of 0.5 g on 2 g of the above-described damaged hairs, then the hairs were dried. Thereafter, the smoothness, the hydrophobicity and the combability were evaluated in the same manners as described above. The results are shown in Table 5.

**[Table 5]**

| | | Smoothness | Hydrophobicity | Combability |
|---|---|---|---|---|
| | | Average friction coefficient | Contact angle (°) | Average load value (N) |
| Example 12 | Diethylaminoethylpalmitamide salt of succinylated polyaspartic acid of Example 5 | 0.88 | 93.2 | 12.48 |
| Example 13 | Diethylaminoethyl-isostearamide salt of maleylated wheat peptide of Example 6 | 0.80 | 97.6 | 11.83 |
| Comparative Example 14 | Diethylaminoethyl-palmitamide salt of polyaspartic acid of Comparative Example 5 | 0.99 | 87.9 | 14.78 |
| Comparative Example 15 | Diethylaminoethylisostearamide salt of wheat peptide of Comparative Example 6 | 0.93 | 91.2 | 13.26 |

As shown in Table 5, hairs treated with the hair mist of the examples 12 to 13 show a larger contact angle, a smaller average friction coefficient and a smaller average load value as compared with hairs treated with the hair mist of the comparative examples 14 to 15. It is understood that the hair mist of the examples 12 to 13 shows a higher effect of imparting hydrophobicity and smoothness to the damaged hair and improving combability than the hair mist of the comparative examples 14 to 15.

### Industrial Applicability

The present invention can provide a cosmetic base material which imparts hydrophobicity and smoothness to the damaged hair and improves the combability of the hair. Additionally, the present invention can provide a cosmetic manifesting the above-described effects by containing this cosmetic base material.

## Claims

1. A cosmetic base material composed of a fatty acid amide amine salt of an acyl peptide obtained by neutralizing part or all of acidic functional groups of an acyl peptide in which a substituent represented by the following general formula (I) : 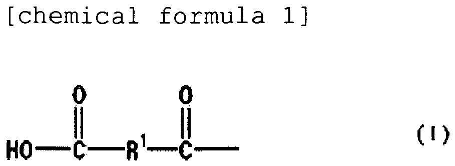 wherein, R¹ represents -CH₂CH₂-, -CH=CH- or -C₆H₄- is linked to an active amino group of a peptide, with an fatty acid amide amine represented by the following general formula (II) : 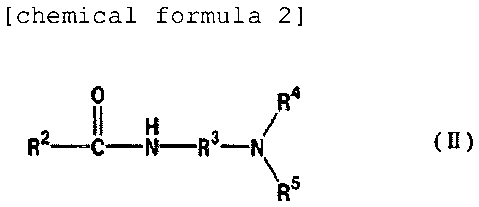 wherein, R² represents a saturated or unsaturated linear hydrocarbon having 11 to 25 carbon atoms or a saturated or unsaturated cyclic hydrocarbon having 11 to 25 carbon atoms, R³ represents an alkylene group having 1 to 3 carbon atoms, and R⁴ and R⁵ represent each independently an alkyl group having 1 to 3 carbon atoms.

2. The cosmetic base material according to Claim 1 wherein the peptide has an acidic amino acid as the constituent amino acid.

3. The cosmetic base material according to Claim 1 or 2 wherein the peptide is a plant protein hydrolysate, a keratin hydrolysate, a casein hydrolysate, polyaspartic acid or polyglutamic acid.

4. The cosmetic base material according to Claim 3 wherein the plant protein hydrolysate is a pea protein hydrolysate.

5. The cosmetic base material according to any one of Claims 1 to 4 wherein the average degree of amino acid polymerization of a peptide part is 2 to 100.

6. The cosmetic base material according to Claim 1 wherein the acyl peptide for forming a salt with a fatty acid amide amine is an acyl peptide represented by the following general formula (III): 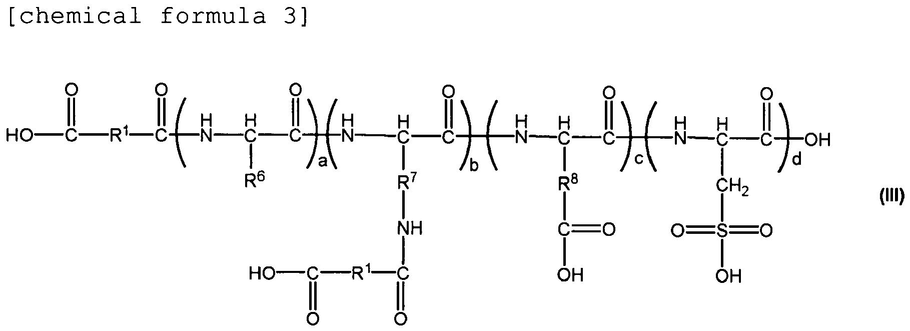 wherein, R¹ is the same as in the above-described general formula (I), R⁶ represents a residue of a side chain at a neutral amino acid, R⁷ represents a residue of a side chain excluding an amino group of a basic amino acid, R⁸ represents an alkylene group having 1 to 2 carbon atoms, a, b, c and d represent the number of each amino acid, a+b+c+d representing the degree of amino acid polymerization, wherein a+b+c+d is 2 or more and c+d is 1 or more, and a, b, c and d represent only the number of an amino acid, and do not represent the order of amino acid sequence.

7. A cosmetic comprising the cosmetic base material according to any one of Claims 1 to 6.

8. The cosmetic according to Claim 7 wherein the content of the above-described cosmetic base material is 0.1 to 10% by mass.

## Patentansprüche

1. Kosmetisches Grundmaterial, das aus einem Fettsäureamidaminsalz eines Acylpeptids besteht, das durch Neutralisieren eines Teils oder der Gesamtheit der sauren funktionellen Gruppen eines Acylpeptids erhalten wird, bei dem ein Substituent durch die folgende allgemeine Formel (I) dargestellt wird: 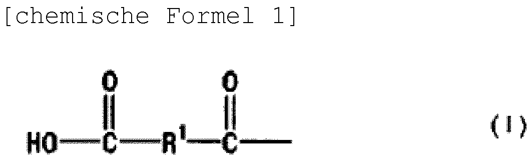 wobei R¹ für -CH₂CH₂-,-CH = CH- oder -C₆H₄- steht und mit einer aktiven Aminogruppe eines Peptids verbunden ist, wobei ein Fettsäureamidamin durch die folgende allgemeine Formel (II) dargestellt wird: 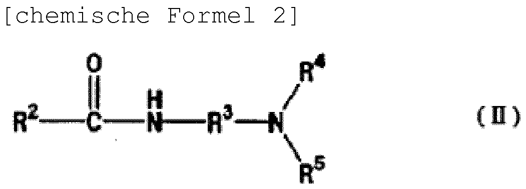 wobei R² für einen gesättigten oder ungesättigten linearen Kohlenwasserstoff mit 11 bis 25 Kohlenstoffatomen oder einen gesättigten oder ungesättigten cyclischen Kohlenwasserstoff mit 11 bis 25 Kohlenstoffatomen steht, R³ für eine Alkylengruppe mit 1 bis 3 Kohlenstoffatomen steht und R⁴ und R⁵ jeweils unabhängig voneinander für eine Alkylgruppe mit 1 bis 3 Kohlenstoffatomen stehen.

2. Kosmetisches Grundmaterial nach Anspruch 1, wobei das Peptid eine saure Aminosäure als konstituierende Aminosäure enthält.

3. Kosmetisches Grundmaterial nach Anspruch 1 oder 2, wobei das Peptid Folgendes ist: ein Pflanzenproteinhydrolysat, ein Keratinhydrolysat, ein Caseinhydrolysat, eine Polyasparaginsäure oder eine Polyglutaminsäure.

4. Kosmetisches Grundmaterial nach Anspruch 3, wobei das Pflanzenproteinhydrolysat ein Erbsenproteinhydrolysat ist.

5. Kosmetisches Grundmaterial nach einem der Ansprüche 1 bis 4, wobei der durchschnittliche Grad der Aminosäurepolymerisation eines Peptidteils 2 bis 100 beträgt.

6. Kosmetisches Grundmaterial nach Anspruch 1, worin das Acylpeptid zur Bildung eines Salzes mit einem Fettsäureamidamin ein Acylpeptid ist, das mit der folgenden allgemeinen Formel (III) dargestellt wird: 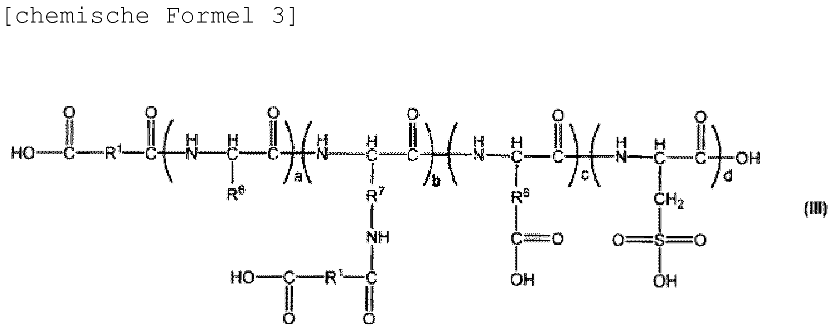 wobei R¹ dem der oben beschriebenen allgemeinen Formel (I) entspricht, R⁶ für einen Rest einer Seitenkette einer neutralen Aminosäure steht, R⁷ für einen Rest einer Seitenkette mit Ausnahme einer Aminogruppe einer basischen Aminosäure steht, R⁸ für eine Alkylengruppe mit 1 bis 2 Kohlenstoffatomen steht, a, b, c und d für die Zahl der einzelnen Aminosäuren stehen, a + b + c + d für den Grad der Aminosäurepolymerisation stehen, wobei a + b + c + d 2 oder mehr ist und c + d 1 oder mehr ist, und a b, c und d nur für die Zahl einer Aminosäure und nicht für die Reihenfolge der Aminosäuresequenz stehen.

7. Kosmetikartikel, der das kosmetische Grundmaterial nach einem der Ansprüche 1 bis 6 umfasst.

8. Kosmetikartikel nach Anspruch 7, wobei der Gehalt des oben beschriebenen kosmetischen Grundmaterials 0,1 bis 10 Massen-% beträgt.

## Revendications

1. Matériau de base cosmétique constitué d'un sel d'amide-amine d'acide gras d'un acyl-peptide obtenu par neutralisation d'une partie ou de la totalité des groupes fonctionnels acides d'un acyl-peptide dans lequel un substituant représenté par la formule générale suivante (I) : 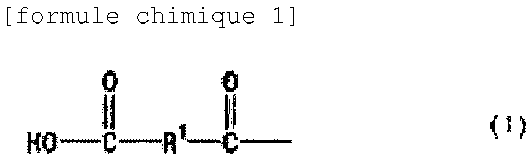 dans laquelle R¹ représente un groupe -CH₂CH₂-, -CH=CHou -C₆H₄-, est lié à un groupe amino actif d'un peptide, avec une amide-amine d'acide gras représentée par la formule générale suivante (II) : 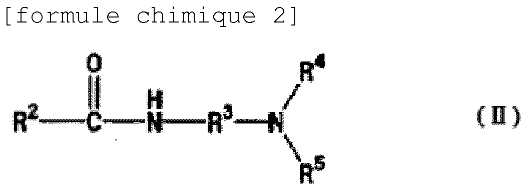 dans laquelle R² représente un groupe hydrocarboné linéaire saturé ou insaturé comportant 11 à 25 atomes de carbone ou un groupe hydrocarboné cyclique saturé ou insaturé comportant 11 à 25 atomes de carbone, R³ représente un groupe alkylène comportant 1 à 3 atomes de carbone, et R⁴ et R⁵ représentent chacun indépendamment un groupe alkyle comportant 1 à 3 atomes de carbone.

2. Matériau de base cosmétique selon la revendication 1, dans lequel le peptide comporte un acide aminé acide comme constituant acide aminé.

3. Matériau de base cosmétique selon la revendication 1 ou 2, dans lequel le peptide est un hydrolysat de protéine végétale, un hydrolysat de kératine, un hydrolysat de caséine, de l'acide polyaspartique ou de l'acide polyglutamique.

4. Matériau de base cosmétique selon la revendication 3, dans lequel l'hydrolysat de protéine végétale est un hydrolysat de protéine de pois.

5. Matériau de base cosmétique selon l'une quelconque des revendications 1 à 4, dans lequel le degré moyen de polymérisation des acides aminés d'une partie peptide est de 2 à 100.

6. Matériau de base cosmétique selon la revendication 1, dans lequel l'acyl-peptide destiné à former un sel avec une amide-amine d'acide gras est un acyl-peptide représenté par la formule générale suivante (III) : 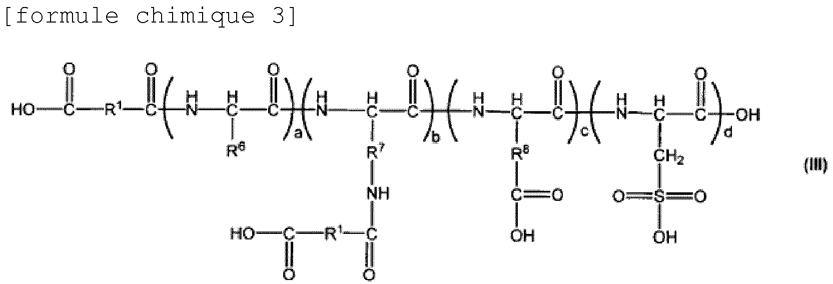 dans laquelle R¹ est le même que dans la formule générale (I) décrite plus haut, R⁶ représente un résidu d'une chaîne latérale d'un acide aminé neutre, R⁷ représente un résidu d'une chaîne latérale à l'exclusion d'un groupe amino d'un acide aminé basique, R⁸ représente un groupe alkylène comportant 1 à 2 atomes de carbone, a, b, c et d représentant le nombre de chaque acide aminé, a+b+c+d représentant le degré de polymérisation des acides aminés, a+b+c+d valant 2 ou plus et c+d valant 1 ou plus, et a, b, c et d représentant seulement le nombre d'un acide aminé, et ne représentant pas l'ordre de la séquence d'acides aminés.

7. Cosmétique comprenant le matériau de base cosmétique selon l'une quelconque des revendications 1 à 6.

8. Cosmétique selon la revendication 7, dans lequel la proportion du matériau de base cosmétique décrit ci-dessus est de 0,1 à 10 % en poids.
